(19) Europäisches Patentamt
European Patent Office
Office européen des brevets

(11) **EP 3 375 363 A1**

(12) **EUROPEAN PATENT APPLICATION**
published in accordance with Art. 153(4) EPC


(43) Date of publication:
**19.09.2018 Bulletin 2018/38**

(21) Application number: **15908310.4**

(22) Date of filing: **12.11.2015**

(51) Int Cl.:
***A61B 5/025*** *(2006.01)*

(86) International application number:
**PCT/JP2015/081859**

(87) International publication number:
**WO 2017/081790 (18.05.2017 Gazette 2017/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**



(71) Applicant: **Fujitsu Limited**
**Kawasaki-shi, Kanagawa 211-8588 (JP)**

(72) Inventors:
- **UCHIDA, Daisuke**
**Kawasaki-shi**
**Kanagawa 211-8588 (JP)**
- **MORI, Tatsuya**
**Kawasaki-shi**
**Kanagawa 211-8588 (JP)**
- **WASHIZAWA, Shiho**
**Kawasaki-shi**
**Kanagawa 211-8588 (JP)**
- **MAEDA, Kazuho**
**Kawasaki-shi**
**Kanagawa 211-8588 (JP)**
- **INOMATA, Akihiro**
**Kawasaki-shi**
**Kanagawa 211-8588 (JP)**

(74) Representative: **Hoffmann Eitle**
**Patent- und Rechtsanwälte PartmbB**
**Arabellastraße 30**
**81925 München (DE)**


# (54) INFORMATION PROCESSING DEVICE, INFORMATION PROCESSING METHOD, AND INFORMATION PROCESSING PROGRAM


(57) An information processing device includes: a heart rate acquiring unit configured to acquire a heart rate of a subject; a feature extracting unit configured to extract a feature of a heart rate in relation to intake of alcohol, from a temporal change in the heart rate acquired by the heart rate acquiring unit; a storage unit configured to store beforehand information about a temporal change in the heart rate in relation to intake of alcohol; and a determining unit configured to determine whether the temporal change in the heart rate acquired by the heart rate acquiring unit is due to intake of alcohol, in accordance with the feature extracted by the feature extracting unit and the information stored in the storage unit.


FIG. 2A

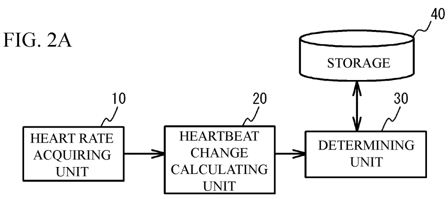


FIG. 2B

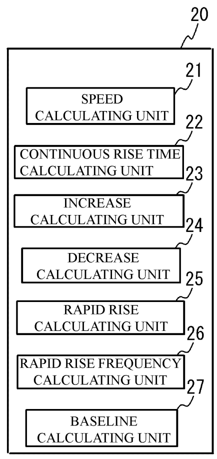


EP 3 375 363 A1

## Description

FIELD

This application relates to an information processing device, an information processing method, and an information processing program.

BACKGROUND

There is a demand for a technology for detecting intake of alcohol beverages. For example, there is a disclosed technology by which a measuring device provided on the driver's seat is used to determine a tendency of chronological variation of the frequency of acquired pulse waves (see Patent Literature 1, for example).

PRIOR ART DOCUMENT

PATENT DOCUMENT

Patent Document 1: International Publication No. 2010/134525

DISCLOSURE OF THE INVENTION

PROBLEMS TO BE SOLVE BY THE INVENTION

However, pulse waves constantly change, and are easily affected by activities such as walking, changes in posture, and psychological changes. Therefore, by the above technology using frequency analysis, it is not possible to achieve high accuracy in alcohol intake detection.

According to an aspect of the present case, provided is an information processing device, an information processing method, and an information processing program that are capable of detecting intake of alcohol with high accuracy.

MEANS FOR SOLVING THE PROBLEMS

In a first aspect, an information processing device includes: a heart rate acquiring unit configured to acquire a heart rate of a subject; a feature extracting unit configured to extract a feature of a heart rate in relation to intake of alcohol, from a temporal change in the heart rate acquired by the heart rate acquiring unit; a storage unit configured to store beforehand information about a temporal change in the heart rate in relation to intake of alcohol; and a determining unit configured to determine whether the temporal change in the heart rate acquired by the heart rate acquiring unit is due to intake of alcohol, in accordance with the feature extracted by the feature extracting unit and the information stored in the storage unit.

EFFECTS OF THE INVENTION

It is possible to detect intake of alcohol under various conditions, independently of a specific action.

BRIEF DESCRIPTION OF DRAWINGS

FIG. 1 is a block diagram showing an example hardware configuration of an information processing device according to a first embodiment.

FIG. 2A is a block diagram of the respective functions to be realized through execution of an information processing program.

FIG. 2B is a functional block diagram showing the respective functions of a heart rate change calculating unit.

FIG. 3 is an example flowchart showing an alcohol intake detecting process.

FIG. 4 is a graph showing an example of a temporal change in heart rate due to intake of alcohol.

FIG. 5 is a graph showing an example of a temporal change in heart rate due to intake of alcohol.

FIG. 6 is an example flowchart showing a feature calculation process.

FIG. 7A is an example flowchart showing the details of step S11 and step S12.

FIG. 7B is an example flowchart showing the details of step S13.

FIG. 7C is an example flowchart showing the details of step S14.

FIG. 8A is an example flowchart showing the details of step S15 and step S16.

FIG. 8B is an example flowchart showing the details of step S51.

FIG. 8C is an example flowchart showing the details of step S17.

FIG. 9 is an example flowchart showing a process of creating models to be stored into a database.

FIG. 10 is an example flowchart of detection of intake of alcohol or non-intake of alcohol.

FIG. 11 is a diagram showing an example of Mahalanobis distances.

FIG. 12 is a block diagram showing an example hardware configuration of an information processing device according to a second embodiment.

FIG. 13 is an example flowchart showing an alcohol intake detecting process.

FIG. 14A is a functional block diagram of an information processing device according to a third embodiment.

FIG. 14B is a functional block diagram showing the respective functions of a heart rate change calculating unit.

FIG. 15 is an example flowchart showing an alcohol intake detecting process.

FIG. 16A shows an example of a temporal change signal s(t) of the heart rate at a time of alcohol intake.

FIG. 16B shows an example of a temporal change signal x(t) of the heart rate acquired in the alcohol intake detecting process.

FIGS. 17A and 17B are diagrams showing other example device configurations of information processing devices.

DESCRIPTION OF EMBODIMENTS

The following is a description of embodiments, with reference to the drawings.

(First Embodiment)

FIG. 1 is a block diagram showing an example hardware configuration of an information processing device 100 according to a first embodiment. As shown in the example in FIG. 1, the information processing device 100 includes a CPU 101, a RAM 102, a storage device 103, a display device 104, a heartbeat measuring device 105, and an acceleration sensor 106. These components are connected by a bus or the like.

The CPU (Central Processing Unit) 101 is a central processor. The CPU 101 includes one or more cores. The RAM (Random Access Memory) 102 is a volatile memory that temporarily stores programs to be executed by the CPU 101, data to be processed by the CPU 101, and the like.

The storage device 103 is a nonvolatile storage device. The storage device 103 may be a read only memory (ROM), a solid state drive (SSD) such as a flash memory, a hard disk to be driven by a hard disk drive, or the like. An information processing program according to this embodiment is stored in the storage device 103. The display device 104 is a liquid crystal display, an electroluminescence panel, or the like, and displays a result of an alcoholic intake detecting process that will be described later.

The heartbeat measuring device 105 is a device that measures the heart rate (pulse rate) of a subject such as a person or an animal that takes alcohol. The heartbeat measuring device 105 is not limited to any particular device, as long as it can measure heart rates (pulse rates). For example, the heartbeat measuring device 105 may be an electrocardiograph or a pulsation sensor, for example. In the description below, the term "heart rate" also means "pulse rate". An alcohol beverage is any beverage containing alcohol, and is not limited to any particular beverage. The acceleration sensor 106 is a sensor that detects the acceleration of an attachment portion attached to the subject.

The information processing program stored in the storage device 103 is loaded into the RAM 102 in an executable manner. The CPU 101 executes the information processing program loaded into the RAM 102. Thus, respective processes are performed by the information processing device 100. FIG. 2A is a block diagram of the respective functions to be realized through execution of the information processing program. As the information processing program is executed, a heart rate acquiring unit 10, a heartbeat change calculating unit 20, a determining unit 30, and a storage unit 40 are formed. In the description below, a storage unit means a database.

FIG. 2B is a functional block diagram showing the respective functions of the heartbeat change calculating unit 20. As shown in the example in FIG. 2B, the heartbeat change calculating unit 20 functions as a speed calculating unit 21, a continuous rise time calculating unit 22, an increase calculating unit 23, a decrease calculating unit 24, a rapid rise calculating unit 25, a rapid rise frequency calculating unit 26, a baseline calculating unit 27, and the like.

(Alcohol Intake Detecting Process)

FIG. 3 is an example flowchart showing the alcohol intake detecting process. As shown in the example in FIG. 3, the heart rate acquiring unit 10 acquires heart rates from the heartbeat measuring device 105, to acquire a temporal change in heart rate (step S1). FIGS. 4 and 5 show examples of temporal changes in heart rate due to alcohol intake. In FIGS. 4 and 5, the abscissa axis indicates elapsed time, and the ordinate axis indicate heart rate. A heart rate is the beating rate per unit time, and, specifically, is the beating rate per minute. Hereinafter, a heart rate means a beating rate per minute, unless otherwise stated. Meanwhile, intake of alcohol is taking alcohol through the mouth more than once within a predetermined time range. In a case where alcohol is newly taken beyond the predetermined time range, it is regarded as the second intake of alcohol. FIGS. 4 and 5 show examples of temporal changes in heart rate due to one-time intake of alcohol.

As shown in the example in FIG. 4, a temporal change in the heart rate due to intake of alcohol has such a feature that the heart rate rises during the intake of alcohol (this feature will be hereinafter referred to as Feature 1). The temporal change also has such a feature that the rise in the heart rate continues during the intake of alcohol (this feature will be hereinafter referred to as Feature 2).

Also, as shown in the example in FIG. 4, the temporal change in the heart rate due to intake of alcohol has such a feature that the temporal change maintains a relatively high value after the end of the intake of alcohol (this feature will be hereinafter referred to as Feature 3).

Further, as shown in the example in FIG. 4, the temporal change in the heart rate due to intake of alcohol has such a feature that the temporal change drops after a body moving activity of the subject, such as walking (this feature will be hereinafter referred to as Feature 4).

In a temporal change in heart rate due to intake of alcohol, variation in the heart rate, or a variance value, for example, becomes larger, due to a sympathetic effect or the like, or motion of intake of alcohol. Therefore, as shown in the example in FIG. 5, the temporal change has such a feature that the heart rate instantaneously has a rapid rise during the intake of alcohol (this feature will be hereinafter referred to as Feature 5). The temporal change also has such a feature that an instantaneous rapid rise in the heart rate occurs at a high frequency during the intake of alcohol (this feature will be hereinafter referred to as Feature 6). The temporal change further has such a feature that the variation of the baseline of the heart rate becomes larger during the intake of alcohol (this feature will be hereinafter referred to as Feature 7). The baseline indicates the temporal change in the heart rate after the above mentioned instantaneous rapid rise is removed.

Therefore, Features 1 through 7 shown in the examples in FIGS. 4 and 5 are stored as a database in advance, so that it is possible to determine whether the subject has taken alcohol by comparing a temporal change in the measured heart rate of the subject with the database. The database includes text files and binary files, and the information to be stored therein is not limited to any particular kind.

Referring back to FIG. 3, the heartbeat change calculating unit 20 calculates the alcohol-intake-related features in the temporal change in the heart rate acquired by the heart rate acquiring unit 10 (step S2). FIG. 6 is an example flowchart showing the feature calculation process. First, since a temporal change in the heart rate due to intake of alcohol has Feature 1 that the heart rate rises during the intake of alcohol, the speed calculating unit 21 calculates the rate of rise in the heart rate as a feature i (step S11). Further, since a temporal change in the heart rate due to intake of alcohol has Feature 2 that the rise of the heart rate continues during the intake of alcohol, the continuous rise time calculating unit 22 calculates the continuous rise time of the heart rate as a feature ii (step S12).

FIG. 7A is a flowchart showing the details of steps S11 and S12. In the temporal change in the heart rate, the speed calculating unit 21 calculates an approximate line by using a time window (step S21). For example, the speed calculating unit 21 uses a temporal change in the heart rate since an alcohol intake start time candidate. The alcohol intake start time candidate can be regarded as the point of time at which a rise of the heart rate is detected. The speed calculating unit 21 then calculates the slope of the approximate line as a heart rate rise rate (step S22).

The continuous rise time calculating unit 22 compares the slope of the approximate line in a preceding time window with the slope of the approximate line in a succeeding time window (step S23). In accordance with a result of the comparison performed in step S23, the continuous rise time calculating unit 22 then calculates the continuous rise time of the heart rate (step S24). For example, in a case where the slope of the approximate line in the succeeding time window becomes smaller than the slope of the approximate line in the preceding time window by an amount equal to or larger than a threshold value, the continuous rise of the heart rate can be determined to have ended. Also, in a case where the ratio of the slope of the approximate line in the succeeding time window to the slope of the approximate line in the preceding time window is equal to or lower than a threshold value, the continuous rise of the heart rate can be determined to have ended. Further, in a case where the slope of the approximate line becomes equal to or smaller than a threshold value, the continuous rise of the heart rate can be determined to have ended. A continuous rise time is the time from an alcohol intake start time candidate till the end of a continuous rise of the heart rate.

The temporal change in the heart rate due to intake of alcohol also has Feature 3 that the temporal change maintains a relatively high value after the end of the intake of alcohol. Therefore, referring back to FIG. 6, the increase calculating unit 23 calculates the increase in the heart rate as a feature iii (step S13). FIG. 7B is an example flowchart showing the details of step S13. After the alcohol intake start time candidate, the increase calculating unit 23 compares the heart rate in the preceding time window with the heart rate in the succeeding time window (step S31). For example, the increase calculating unit 23 calculates the average value between the heart rate at the alcohol intake start time candidate and the heart rate in a time window where the slope of the approximate line is equal to or smaller than a threshold value. In accordance with a result of the comparison performed in step S31, the increase calculating unit 23 then calculates the increase in the heart rate (step S32).

The temporal change in the heart rate due to intake of alcohol also has Feature 4 that the temporal change drops after a body moving activity of the subject, such as walking. Therefore, referring back to FIG. 6, the decrease calculating unit 24 calculates a decrease in the heart rate after an activity such as walking, as a feature iv (step S14). FIG. 7C is an example flowchart showing the details of step S14. Using a result of detection performed by the acceleration sensor 106, the decrease calculating unit 24 detects walking of the subject (step S41). For example, in a case where the degree of coincidence between the pattern of the acceleration detected by the acceleration sensor 106 and the pattern of the acceleration caused by walking is equal to or higher than a threshold value, the subject can be determined to have walked.

The decrease calculating unit 24 then acquires the heart rate after the walking is detected, from the heart rate acquiring unit 10 (step S42). The decrease calculating unit 24 then calculates a difference between the heart rate immediately before the detection of the walking and the heart rate after the detection of the walking, as a decrease in the heart rate (step S43). For example, the decrease calculating unit 24 calculates a difference between the average value of the heart rate in a time window where the slope of the above mentioned approximate line is equal to or smaller than a threshold value and the average value of the heart rate in a case where the drop rate of the heart rate becomes equal to or lower than a threshold value after the drop rate of the heart rate became equal to or higher than the threshold value after the detection of the walking. The difference is regarded as a decrease in the heart rate.

The temporal change in the heart rate due to intake of alcohol also has Feature 5 that the heart rate instantaneously has a rapid rise during the intake of alcohol. Therefore, referring back to FIG. 6, the rapid rise calculating unit 25 calculates a rapid rise in the heart rate and a rapid rise time as features v and vi (step S15). A rapid rise in the heart rate can be regarded as a zone in which the heart rate increases or decreases by a predetermined amount, such as one beat per minute, within a predetermined time such as 15 minutes. The heart rate may be an average value in a predetermined time such as five minutes. Alternatively, the predetermined time of a rapid rise in the heart rate may be a zone in which the heart rate increases or decreases within one minute. The temporal change in the heart rate due to intake of alcohol also has Feature 6 that an instantaneous rapid rise in the heart rate occurs at a high frequency during intake of alcohol. Therefore, the rapid rise calculating unit 25 calculates the frequency of occurrence of a rapid rise pattern of the heart rate or the variance value of the heart rate, or the variation of intervals between the peaks in a rapid rise pattern, as a feature vii (step S16). A rapid rise pattern of the heart rate is the zone calculated in step S15, and the number of occurrences of rapid rise patterns in a predetermined time such as one hour is regarded as the occurrence frequency. Likewise, the variance value of the heart rate can be regarded as the variance value of the change in the heart rate in a predetermined time such as one hour. Further, the variation of intervals between the peaks can be regarded as the variation of peak intervals in a predetermined time such as one hour in a case where the maximum value in a rapid rise pattern zone is regarded as a peak value. FIG. 8A is an example flowchart showing the details of steps S15 and 16. The rapid rise calculating unit 25 first calculates heart rate rapid rise zones (step S51).

FIG. 8B is an example flowchart showing the details of step S51. As shown in the example in FIG. 8B, the rapid rise calculating unit 25 calculates the lowest heart rate, using a time window (step S61). The rapid rise calculating unit 25 then compares the heart rate at the respective measurement points in the temporal change in the heart rate with the lowest heart rate, to calculate a heart rate difference (step S62). Using a result of the comparison performed in step S62, the rapid rise calculating unit 25 then identifies a rapid rise pattern of the heart rate (step S63). For example, in a case where the heart rate difference calculated in step S62 appears within a predetermined time in a rapid rise pattern, the heart rate can be determined to have a rapid rise. A zone in which a rapid rise pattern appears is a rapid rise zone. Instead of the lowest heart rate, a smoothed (averaged) heart rate within a predetermined time may be used.

Referring back to FIG. 8A, the rapid rise calculating unit 25 then calculates the heart rates before and after rapid rise patterns (step S52). Using differences between the result of the calculation in step S52 and the peaks of the respective rapid rise patterns, the rapid rise calculating unit 25 calculates the rises in the respective rapid rise patterns (step S53). The rapid rise calculating unit 25 then calculates the lengths of times of the respective rapid rise patterns (step S54). The rapid rise calculating unit 25 then calculates the rapid rise frequency, using the time window (step S55). The rapid rise calculating unit 25 then calculates the variance value of the rapid rises in the heart rate, using the time window (step S56). The rapid rise calculating unit 25 then calculates the variation of the time intervals between the peaks of the rapid rise patterns (step S57).

The temporal change in the heart rate due to intake of alcohol also has Feature 7 that the variation of the baseline of the heart rate becomes larger during the intake of alcohol. Therefore, referring back to FIG. 6, the baseline calculating unit 27 calculates a baseline variance value as a feature viii (step S17). FIG. 8C is a flowchart showing the details of step S17. As shown in the example in FIG. 8C, the baseline calculating unit 27 calculates a baseline by removing the heart rate rapid rise patterns, using the result of step S51 in FIG. 8A (step S71). The baseline calculating unit 27 then calculates a baseline variance value, using the time window (step S72).

The database 40 stores models that have been created in advance. The models include a model related to intake of alcohol and a model related to non-intake of alcohol. The model related to intake of alcohol includes the features i through viii in the temporal change in the heart rate due to intake of alcohol. The model related to non-intake of alcohol includes the features i through viii in the temporal change in the heart rate during a time in which no alcohol is taken. These models may be created with the use of actual measured values of a specific subject using the alcohol intake detecting process, or may be created with the use of actual measured values of a large number of unidentified users. In a case where the measured values in two or more operations are used, the average values in the operations may be used. Alternatively, the subject may create the model of a time at which alcohol is taken and the model of a time at which no alcohol is taken.

FIG. 9 is an example flowchart showing a process of creating models to be stored into the database 40. This example is an example where models are created beforehand with the use of measured values of the heart rate of a subject using the alcohol intake detecting process. As shown in the example in FIG. 9, the heart rate acquiring unit 10 sequentially reads heart rate data accompanied by labels (step S81). The labels can be created by pressing a button or the like every time the subject takes alcohol.

The heartbeat change calculating unit 20 then calculates the features related to rises and drops in the temporal change in the heart rate (step S82). The features include the features i through viii. The heartbeat change calculating unit 20 then stores the calculated features together with the labels as a database for model creation into the database 40 (step S83). The determining unit 30 then determines whether the process has been completed for all the data (step S84). In a case where the determination result is "No" in step S84, the process is repeated from step S81. In a case where the determination result is "Yes" in step S84, the determining unit 30 creates a model related to intake of alcohol and a model related to non-intake of alcohol, using the database for model creation (step S85). These models are stored as the models to be used in the alcohol intake detecting process into the database 40.

Referring back to FIG. 3, in accordance with the features calculated by the heartbeat change calculating unit 20, the determining unit 30 determines whether the change in the heart rate at the time of the alcohol intake detecting process has been caused by intake of alcohol (step S3). FIG. 10 is an example flowchart showing the determination process in this case. As shown in the example in FIG. 10, the determining unit 30 calculates the degree of coincidence (or the degree of inconsistency) between the features obtained from the temporal change in the heart rate measured at the time of the alcohol intake detecting process, and the alcohol intake related model and the alcohol non-intake related model stored in the database 40 (step S91). The degree of coincidence indicates the degree of sameness between the features and the models, and is synonymous with a degree of similarity or a degree of resemblance. Although Mahalanobis distances are used as a coincidence calculation method in the description below, it is also possible to use any appropriate algorithm of the support vector machine, boosting, a neural network, or the like.

As an example, a case where the determining unit 30 uses Mahalanobis distances in calculating the degree of coincidence (the degree of inconsistency) is described. When calculating a Mahalanobis distance, the determining unit 30 uses a mean vector and a covariance matrix as parameters. The mean vector and the covariance matrix in a case where alcohol is taken are represented by $\mu_d$ and $\Sigma_d$, the mean vector and the covariance matrix in a case where no alcohol is taken are represented by $\mu_n$ and $\Sigma_n$, and the data to be determined is $x = (x_1, x_2, ...)^T$. Here, $x_1$, $x_2$, ... represent the feature i, the feature ii, ..., respectively. The Mahalanobis distance can be expressed by the equation (1) and the equation (2) shown below. In a case where the covariance matrix is a diagonal matrix, a normalized Euclidean distance (on which normalization has been performed so that the variances of the features become equal) may be used. In the case of an identity matrix, an Euclidean distance may be used.

[Expression 1]

$$D_d = \sqrt{(x-\mu_d)^T \Sigma_d^{-1} (x-\mu_d)} \quad (1)$$

[Expression 2]

$$D_n = \sqrt{(x-\mu_n)^T \Sigma_n^{-1} (x-\mu_n)} \quad (2)$$

The determining unit 30 then determines that the heart rate change measured at the time of the alcohol intake detecting process belongs to a short distance group (a high coincidence group) (step S92). Therefore, if $D_d > D_n$ as shown in the example in FIG. 11, the determining unit 30 determines that the heart rate change measured at the time of the alcohol intake detecting process is a heart rate change due to non-intake of alcohol. For ease of explanation, FIG. 11 shows examples of distances, using only the features i and ii. Alternatively, information about the viscosity of liquid and the existence/nonexistence of solid matter is estimated with the use of the values of $D_d$ and $D_n$, and may be added to the result of the determination performed by the determining unit 30. For example, in a case where $D_d < D_n$, and the ratio between the $D_d$ and $D_n$ is equal to or lower than a predetermined value (2, for example), the determining unit 30 determines that the heart rate change measured at the time of the alcohol intake detecting process is the heart rate change at a time of intake of a beverage containing solid matter. Alternatively, the distances from models at times of intake of alcohol, instead of the model at a time of non-intake of alcohol, may be calculated, and the concentration of alcohol may be estimated and added to the result of the determination performed by the determining unit 30. For example, alcohol concentrations are estimated from the distances from a model at a time of intake of a beverage with a low alcohol concentration and a model at a time of intake of a beverage with a high alcohol concentration, and the heart rate changes are determined to be those due to intake of a beverage with an alcohol concentration of 5% and due to intake of a beverage with an alcohol concentration of 20%.

According to this embodiment, a check is made to determine whether a temporal change in the heart rate of a subject has been caused by intake of alcohol, in accordance with the degree of coincidence between the features extracted from the temporal change in the heart rate of the subject and the alcohol-intake-related features stored in a database. Thus, it is possible to detect intake of alcohol simply by acquiring a temporal change in the heart rate. Since only the temporal change in the heart rate should be measured, there is no need to take any particular action before and after intake of alcohol. Further, with the use of a wearable device attached to an arm or the like, intake of alcohol can be detected in a simple manner with low load. Since an acceleration sensor or the like is provided, it is possible to detect a change in the heart rate due to an action of the subject. Thus, the accuracy of alcohol intake detection increases.

In a case where the degree of coincidence with the features that are related to intake of alcohol and are stored in the database is higher than the degree of coincidence with the features that are related to non-intake of alcohol and are stored in the database, the temporal change in the heart rate is determined to be due to intake of alcohol. Thus, the accuracy of alcohol intake detection increases. However, the degree of coincidence with the features related to non-intake of alcohol is not necessarily calculated. For example, if the degree of coincidence with the features that are related to intake of alcohol and are stored in the database is equal to or higher than a threshold value, the temporal change in the heart rate may be determined to be due to intake of alcohol. For example, in step S92 in FIG. 10, the degree of coincidence may be compared with the threshold value. Although at least one of the above described features are required, the use of the features i, ii, v, vi, vii, and viii will further increase the accuracy of alcohol intake detection.

With a combination of the result of determination performed by the determining unit 30 and information about behaviors such as exercise and medication, it becomes possible to give advice on daily habits. For example, in a case where it is known beforehand that the subject takes medicine at a predetermined time, or an action of taking medicine is detected, a comment "please refrain from drinking alcohol when you are on mediation" or the like is displayed. The result of the determination may include at least one of the following: the number of alcohol intake times, an alcohol intake start time, an alcohol intake end time, and an amount of intake of alcohol. The number of alcohol intake times is equivalent to the number of times intake of alcohol has been detected in a predetermined period such as one day. The alcohol intake start time is equivalent to an alcohol intake start time candidate, for example. The alcohol intake end time is equivalent to a time at which the average value of the heart rate in a time window where the slope of the approximate line of the heart rate is equal to or smaller than a threshold value is equal to or greater than a predetermined value. The amount of intake of alcohol is equivalent to the value of a heart rate rise area calculated from the relationship among the heart rate rise time, the rise amount, and the blood-alcohol content in the period from an alcohol intake start time to an alcohol intake end time that are determined in advance.

(Second Embodiment)

A second embodiment concerns an example where behavioral information about the subject is reflected in the alcohol intake detecting process. FIG. 12 is a block diagram showing an example hardware configuration of an information processing device 100a according to the second embodiment. As shown in the example in FIG. 12, the information processing device 100a includes a behavioral information detecting device 106a, instead of the acceleration sensor 106. The behavioral information detecting device 106a is a device that detects behavioral information about the subject.

The behavioral information detecting device 106a detects behaviors having low correlations with intake of alcohol. For example, the behavioral information detecting device 106a detects behaviors that physically hinder intake of alcohol. Specifically, the behavioral information detecting device 106A detects the subject's sleeping, vocalizing, masticating, sighing, and the like. The behavioral information detecting device 106a also detects behaviors with which the subject is highly likely to take no alcohol. Specifically, the behavioral information detecting device 106a detects general behaviors. For example, the behavioral information detecting device 106a detects behaviors while the subject is standing and moving his/her legs, such as walking, running, climbing stairs, and the like. The behavioral information detecting device 106a also detects behaviors while the subject is moving his/her hands, such as washing dishes, cleaning, and the like. The behavioral information detecting device 106a also detects behaviors for riding a certain vehicle such as a motorcycle. The behavioral information detecting device 106a also detects behaviors related to excretion. The behavioral information detecting device 106a also detects behaviors related to bathing. The behavioral information detecting device 106a also detects behaviors related to medication. The behavioral information detecting device 106a also detects individual behaviors. For example, the behavioral information detecting device 106a detects behaviors such as having a conversation with a specific person, attending a meeting, or being in a situation where one does not want to have an urge to urinate, such as standing in a line.

Alternatively, the behavioral information detecting device 106a also detects behaviors having high correlations with intake of alcohol. For example, the behavioral information detecting device 106a detects behaviors specific to intake of alcohol (actions of body parts having slopes that vary with intake of alcohol, such as the arms and the chest). Specifically, the behavioral information detecting device 106a detects preparatory actions for pouring liquid into a glass (an action of tilting a glass in a case where the subject is pouring beer into the glass, for example), movement from front to back or vertical movement at a time when a glass is brought to the mouth, a resting state for a predetermined period during which the glass is brought to the mouth and the subject drinks the content, movement backward or downward when the subject finishes the drink in the glass, and movement from back to front or vertical movement when the glass is returned onto the table or the like. The behavioral information detecting device 106a also detects places specific to intake of alcohol. For example, the behavioral information detecting device 106a detects restaurants, bars, the subject's residence, and the like. The behavioral information detecting device 106a also detects behaviors with which the subject is highly lightly to take alcohol. For example, the behavioral information detecting device 106a detects general habitual behaviors. For example, the behavioral information detecting device 106a detects a habit of drinking alcohol before or after taking a bath, a habit of drinking alcohol after exercise, a habit of drinking alcohol during a meal, a habit of drinking alcohol at a certain hour (such as 21:00 or after getting home), a habit of drinking alcohol on the way home, and a habit of drinking alcohol at a specific place (such as in a specific chair). Identifying behaviors from action data, or identifying times and places from data related to times and places may be performed by an existing technology. For example, a timer, an acceleration sensor, a GPS function, and the like may be used to detect behavioral information. Alternatively, it is possible to acquire labels created by pressing a button or the like every time the subject takes any of the actions.

FIG. 13 is an example flowchart showing the alcohol intake detecting process according to this embodiment. As shown in the example in FIG. 13, the heart rate acquiring unit 10 acquires a heart rate from the heartbeat measuring device 105, to acquire a temporal change in the heart rate (step S101). In a case where the behavioral information detecting device 106a detects a behavior of the subject, the heart rate acquiring unit 10 acquires behavioral information about the subject in conjunction with the temporal change in the heart rate. The heartbeat change calculating unit 20 then calculates the features related to intake of alcohol in the temporal change in the heart rate acquired by the heart rate acquiring unit 10 (step S102).

The determining unit 30 then calculates the Mahalanobis distances between the features calculated by the heartbeat change calculating unit 20, and a model related to intake of alcohol and a model related to non-intake of alcohol (step S103). The determining unit 30 then calculates the ratio between the respective distances (step S104). The ratio may be the ratio of the distance from the model related to intake of alcohol to the distance from the model related to non-intake of alcohol, for example.

If the temporal change in the heart rate acquired by the heart rate acquiring unit 10 includes action data of the subject, the determining unit 30 identifies the behavior related to the action data (step S105). If the temporal change in the heart rate acquired by the heart rate acquiring unit 10 includes data related to time and the location of the subject, the determining unit 30 identifies the time and the location (step S106).

The determining unit 30 then refers to the threshold values stored beforehand in the database 40, and acquires the threshold value associated with the behavior detected by the behavioral information detecting device 106a. If the distance ratio calculated in step S104 is equal to or lower than the threshold value, the determining unit 30 determines that the subject has not taken alcohol (step SI07). The determining unit 30 then determines whether the measurement has ended (step S108). If the determination result in step S108 is "No", the process is repeated from step S101. If the determination result in step S108 is "Yes", the execution of the flowchart comes to an end.

According to this embodiment, when a check is made to determine whether a temporal change in the heart rate of the subject is due to intake of alcohol, the behavioral information about the subject is reflected in the determination. Thus, the accuracy of alcohol intake determination increases. For example, in a case where there is a low probability of intake of alcohol, the threshold for the distance ratio calculated in step S104 is made smaller, so that false detection of intake of alcohol can be prevented. In a case where there is a high probability of intake of alcohol, the threshold value for the distance ratio calculated in step S104 is made greater, so that false detection of non-intake of alcohol can be prevented.

(Third Embodiment)

FIG. 14A is a functional block diagram of an information processing device 100b according to a third embodiment. The functions are realized through execution of an information processing program. This embodiment differs from the first embodiment in further including an internal function quantifying unit 50. FIG. 14B is a functional block diagram showing the respective functions of the heartbeat change calculating unit 20. The respective functions of the heartbeat change calculating unit 20 are the same as those of the first embodiment. The internal function quantifying unit 50 quantifies internal functions by comparing features obtained from the temporal change in the heart rate acquired at the time of the alcohol intake detecting process with a model related to intake of alcohol. For example, if the rise rate of the acquired heart rate is twice higher than the model rise rate, the internal function quantifying unit 50 determines that the subject is twice thirstier than usual. If the drop rate of the acquired heart rate is 1/2 of the model drop rate, the internal function quantifying unit 50 determines that the body of the subject is twice weaker than usual. If the increase in the acquired heart rate is 1.5 times greater than the model increase, the internal function quantifying unit 50 determines that the subject is 1.5 times drunker than usual. Using a walking situation or a state of walking, the internal function quantifying unit 50 can further increase the accuracy of drunkenness detection. Also, by comparing a feature with a feature of another person, such as comparing the increase in the heart rate of the subject with the increase in the heart rate of some other person, the subject can be determined to have a higher or lower degree of drunkenness than the other person.

FIG. 15 is an example flowchart showing the alcohol intake detecting process according to this embodiment. As shown in the example in FIG. 15, the heart rate acquiring unit 10 acquires a heart rate from the heartbeat measuring device 105, to acquire a temporal change in the heart rate (step S111). The heartbeat change calculating unit 20 then calculates the features related to intake of alcohol in the temporal change in the heart rate acquired by the heart rate acquiring unit 10 (step S112). Using the features calculated in step S112, the determining unit 30 determines whether the subject has taken alcohol or has not taken alcohol (step S113).

The internal function quantifying unit 50 then determines whether the result of the determination in step S113 is "intake of alcohol" (step S114). If the result of the determination in step S114 is "Yes", the internal function quantifying unit 50 refers to the database 40, and acquires the respective features of the model at a time of the alcohol intake (step S115). In a case where a feature in the model at a time of intake of alcohol includes more than one value, the average value thereof may be acquired.

The internal function quantifying unit 50 then calculates the ratios between the respective features calculated in step S112 and the respective features acquired in step S115 (step S116). The internal function quantifying unit 50 then quantifies thirstiness, in accordance with the rise rate of the heart rate (step S117). The internal function quantifying unit 50 then quantifies the body function, in accordance with the drop rate of the heart rate. The internal function quantifying unit 50 further quantifies drunkenness, in accordance with the increase in the heart rate (step S118). The internal function quantifying unit 50 then determines whether the measurement has ended (step S119). If the determination result is "Yes" in step S119, the execution of the flowchart comes to an end. If the determination result in step S119 is "No", the process is repeated from step S111. In a case where the determination result is "No" in step S114, step S119 is also carried out.

According to this embodiment, the internal functions of the subject can be quantified, with the use of the relationship between the features in a temporal change in the heart rate of the subject and the features stored in a database.

(Other Features)

In each of the above examples, features such as the rise rate in a temporal change in the heart rate are used, but features are not limited to them. For example, the waveform pattern of a temporal change in the heart rate may be used as a feature. Specifically, in a case where the degree of coincidence (the degree of inconsistency) between the waveform pattern of the heart rate of the subject and an alcohol-intake-related waveform pattern related stored beforehand in the database 40 is high, intake of alcohol may be detected.

A temporal change signal of a heart rate related to intake of alcohol registered in the database 40 is represented by s(t), and a temporal change signal of the heart rate acquired at the time of an alcohol intake detecting process is represented by x(t). FIG. 16A shows an example of s(t). FIG. 16B shows an example of x(t). In this case, the degree of coincidence between the two patterns can be calculated according to the equation (3) shown below. In the equation (3), the difference is calculated. The smaller the value of the difference, the higher the degree of coincidence. N represents the total number of signals. The number of signals is the number of seconds from the time of the start of the period before a rise till the time of the end of the period after the drop. In a case where the degree of coincidence is equal to or higher than a predetermined threshold value, intake of a beverage is detected. In a case where the number of signals differs from the total number N of signals, the signals should be interpolaed or reduced.

[Expression 3]

$$\|s - x\| = \sqrt{\sum_{j=1}^{N} \left(s(t_j) - x(t_j)\right)^2} \qquad (3)$$

(Other Device Configurations)

FIGS. 17A and 17B are diagrams showing other example device configurations of the information processing device 100. As shown in the example in FIG. 17A, an information processing device may have a configuration in which data is wirelessly exchanged between a server including a CPU 101, a RAM 102, a storage device 103, and a wireless device 107, and a wearable device including a display device 104, a heartbeat measuring device 105, an acceleration sensor 106, and a wireless device 108. Alternatively, as shown in the example in FIG. 17B, an information processing device may have a configuration in which data is wirelessly exchanged among a server including a CPU 101, a RAM 102, a storage device 103, and a wireless device 107, a terminal including a display device 104 and a wireless device 108, and a wearable device including a heartbeat measuring device 105, an acceleration sensor 106, and a wireless device 109. In a case where a behavioral information detecting device 106a is provided, the wearable device may include the behavioral information detecting device 106a.

In the above embodiments, the heart rate acquiring unit 10 functions as an example of the heart rate acquiring unit that acquires the heart rate of the subject. The heartbeat change calculating unit 20 functions as an example of the feature extracting unit that extracts features from a temporal change in the heart rate acquired by the heart rate acquiring unit. The database 40 functions as an example of the database that stores beforehand the features extracted from a temporal change of the heart rate in relation to intake of alcohol. The determining unit 30 functions as an example of the determining unit that determines whether a temporal change in the heart rate acquired by the heart rate acquiring unit is due to intake of alcohol, in accordance with the degree of coincidence between the features extracted by the feature extracting unit and the features stored in the database. The behavioral information detecting device 106a functions as an example of the behavioral information measuring device that measures behavioral information about the subject. The internal function quantifying unit 50 functions as an example of the quantifying unit that quantifies the internal functions of the subject in accordance with the relationship between the features extracted by the feature extracting unit and the features stored in the database, in a case where the determining unit determines that the temporal change in the heart rate acquired by the heart rate acquiring unit is due to intake of alcohol.

Although embodiments of the present invention have been described so far, the present invention is not limited to these specific embodiments, and various changes and modifications may be made to them within the scope of the claimed invention.

[Reference Signs List]

10 Heart rate acquiring unit
20 Heartbeat change calculating unit
21 Speed calculating unit
22 Continuous rise time calculating unit
23 Increase calculating unit
24 Decrease calculating unit
25 Rapid rise calculating unit
26 Rapid rise frequency calculating unit
27 Baseline amount calculating unit
30 Determining unit
40 Storage unit
50 Internal function quantifying unit
100 Information processing device
105 Heartbeat measuring device
106 Acceleration sensor
106a Behavioral information detecting device

## Claims

1. An information processing device **characterized by** comprising:

   a heart rate acquiring unit configured to acquire a heart rate of a subject;
   a feature extracting unit configured to extract a feature of a heart rate in relation to intake of alcohol, from a temporal change in the heart rate acquired by the heart rate acquiring unit;
   a storage unit configured to store beforehand information about a temporal change in the heart rate in relation to intake of alcohol; and
   a determining unit configured to determine whether the temporal change in the heart rate acquired by the heart rate acquiring unit is due to intake of alcohol, in accordance with the feature extracted by the feature extracting unit and the information stored in the storage unit.

2. The information processing device according to claim 1, **characterized in that**, when a degree of coincidence between the feature extracted by the feature extracting unit and a feature extracted from the information stored in the storage unit is not lower than a threshold value, the determining unit determines that the temporal change in the heart rate acquired by the heart rate acquiring unit is due to intake of alcohol.

3. The information processing device according to claim 1, **characterized in that**:

   the storage unit is configured to store beforehand information about a temporal change in a heart rate in relation to non-intake of alcohol; and
   when a degree of coincidence between the feature extracted by the feature extracting unit and a feature in a heart rate extracted from the information stored in the storage unit in relation to intake of alcohol is higher than a degree of coincidence between the feature extracted by the feature extracting unit and a feature in a heart rate extracted from the information stored in the storage unit in relation to non-intake of alcohol, the determining unit determines that the temporal change in the heart rate acquired by the heart rate acquiring unit is due to intake of alcohol.

4. The information processing device according to any of claims 1 to 3, **characterized in that** the degree of coincidence is calculated in accordance with one of an Euclidean distance, an Euclidean normalized distance, and a Mahalanobis distance.

5. The information processing device according to any of claims 1 to 4, **characterized by** further comprising a behavioral information measuring device configured to measure behavioral information about the subject,
   wherein the determining unit is configured to cause the behavioral information to be reflected in determining whether the temporal change in the heart rate acquired by the heart rate acquiring unit is due to intake of alcohol, the behavioral information being measured by the behavioral information measuring device.

6. The information processing device according to any of claims 1 to 5, **characterized by** further comprising a quantifying unit configured to quantify an internal function of the subject in accordance with a relationship between the feature extracted by the feature extracting unit and a feature extracted from the information stored in the storage unit, when the determining unit determines that the temporal change in the heart rate acquired by the heart rate acquiring unit is due to intake of alcohol.

7. The information processing device according to any of claims 1 to 6, **characterized in that** the feature includes a rise rate of the heart rate.

8. The information processing device according to any of claims 1 to 6, **characterized in that** the feature includes a time for which the heart rate continues to rise.

9. The information processing device according to any of claims 1 to 6, **characterized in that** the feature includes an increase in the heart rate.

10. The information processing device according to any of claims 1 to 6, **characterized by** further comprising a detecting device configured to detect walking of the subject,
wherein the feature includes a decrease in the heart rate since walking of the subject was detected by the detecting device.

11. The information processing device according to any of claims 1 to 6, **characterized in that** the feature includes an increase/decrease in the heart rate in a zone in which the heart rate increases and decreases within a predetermined time, and a time length of the zone.

12. The information processing device according to any of claims 1 to 6, **characterized in that** the feature includes at least one of a frequency of occurrence of a rapid rise pattern in which the heart rate increases and decreases within a predetermined time and a variance value of the heart rate, and variation of time intervals between peaks of rapid rise patterns.

13. The information processing device according to any of claims 1 to 6, **characterized in that** the feature includes a variance value of a baseline after variation of the heart rate in the zone is removed from the temporal change in the heart rate to be acquired by the heart rate acquiring unit.

14. The information processing device according to any of claims 1 to 6, **characterized in that** the feature includes a waveform pattern of the temporal change in the heart rate.

15. The information processing device according to any of claims 1 to 6, **characterized in that** the feature includes at least one of a rise rate of the heart rate, a time for which the heart rate continues to rise, an increase in the heart rate, a decrease in the heart rate since walking of the subject was detected by a detecting device designed to detect walking of the subject, an increase/decrease in the heart rate in a zone in which the heart rate increases and decreases by a predetermined amount within a predetermined time and a time length of the zone, a frequency of occurrence of a rapid rise pattern in which the heart rate increases and decreases within a predetermined time, a variance value of the heart rate, and variation of time intervals between peaks of rapid rise patterns, a variance value of a baseline after variation of the heart rate during the zone is removed from the temporal change in the heart rate to be acquired by the heart rate acquiring unit, and a waveform pattern of the temporal change in the heart rate.

16. The information processing device according to any of claims 1 to 6, **characterized in that** the feature includes a rise rate of the heart rate, a time for which the heart rate continues to rise, an increase/decrease in the heart rate in a zone in which the heart rate increases and decreases by a predetermined amount within a predetermined time and a time length of the zone, a frequency of occurrence of a rapid rise pattern in which the heart rate increases and decreases within a predetermined time, a variance value of the heart rate, and variation of time intervals between peaks of rapid rise patterns, and a variance value of a baseline after variation of the heart rate in the zone is removed from the temporal change in the heart rate to be acquired by the heart rate acquiring unit.

17. An information processing method comprising:

acquiring a heart rate of a subject, a heart rate acquiring unit performing the acquisition;
extracting a feature of a heart rate in relation to intake of alcohol from a temporal change in the heart rate acquired by the heart rate acquiring unit, a feature extracting unit performing the extraction;
storing beforehand information about a temporal change in the heart rate in relation to intake of alcohol, a storage unit performing the storing; and
determining whether the temporal change in the heart rate acquired by the heart rate acquiring unit is due to intake of alcohol, in accordance with the feature extracted by the feature extracting unit and the information stored in the storage unit, a determining unit performing the determination.

18. An information processing program for causing a computer to perform:

a process of acquiring a heart rate of a subject;
a process of extracting a feature of a heart rate from a temporal change in the acquired heart rate;
a process of storing beforehand information about a temporal change in a heart rate in relation to intake of alcohol, into a storage unit; and
a process of determining whether the temporal change in the heart rate of the subject is due to intake of alcohol, in accordance with the feature extracted through the process of extracting the feature and the information stored in the storage unit.

FIG. 1

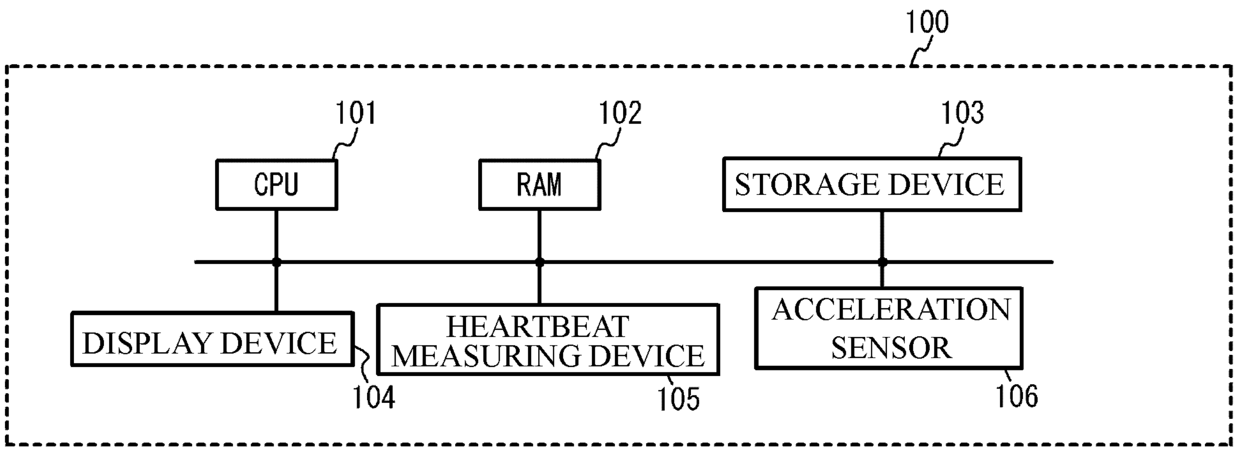
100
101
CPU
102
RAM
103
STORAGE DEVICE
DISPLAY DEVICE
104
HEARTBEAT
MEASURING DEVICE
105
ACCELERATION
SENSOR
106

FIG. 2A

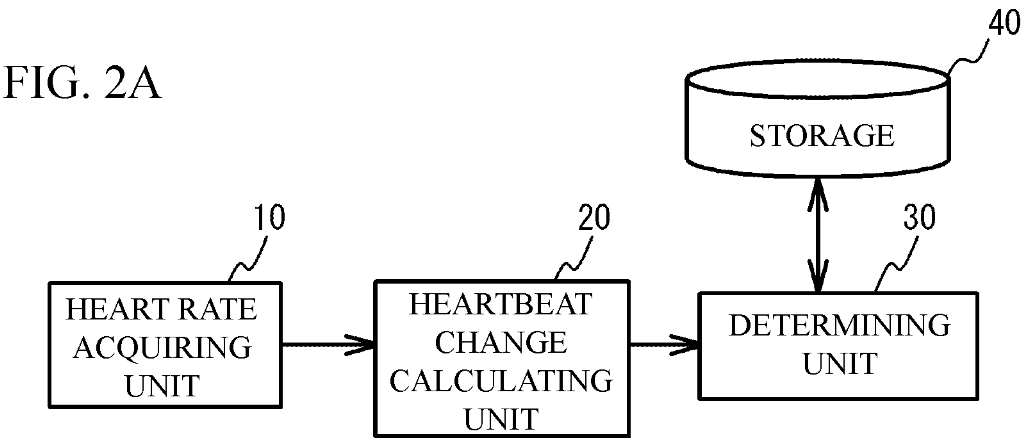
40
STORAGE
10
20
30
HEART RATE ACQUIRING UNIT
HEARTBEAT CHANGE CALCULATING UNIT
DETERMINING UNIT

FIG. 2B

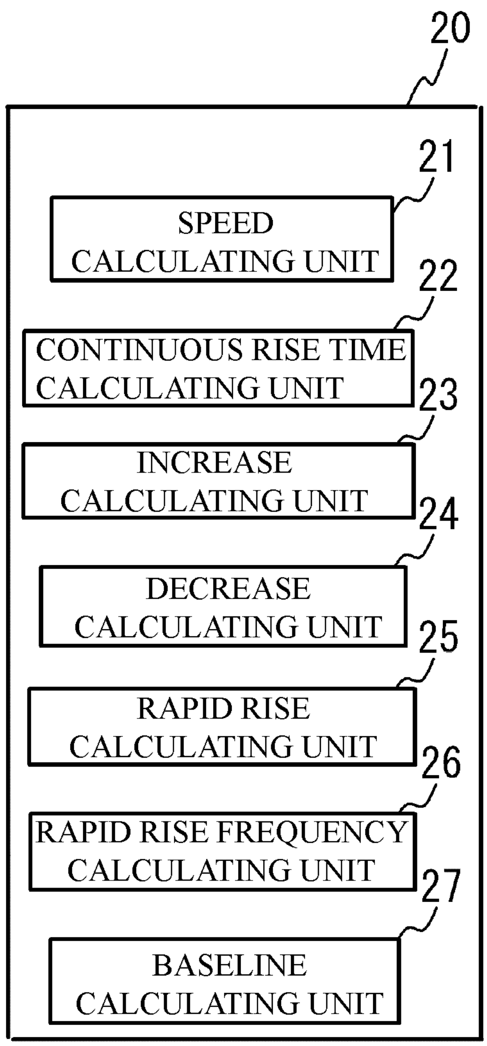
20
21
SPEED CALCULATING UNIT
22
CONTINUOUS RISE TIME CALCULATING UNIT
23
INCREASE CALCULATING UNIT
24
DECREASE CALCULATING UNIT
25
RAPID RISE CALCULATING UNIT
26
RAPID RISE FREQUENCY CALCULATING UNIT
27
BASELINE CALCULATING UNIT

FIG. 3

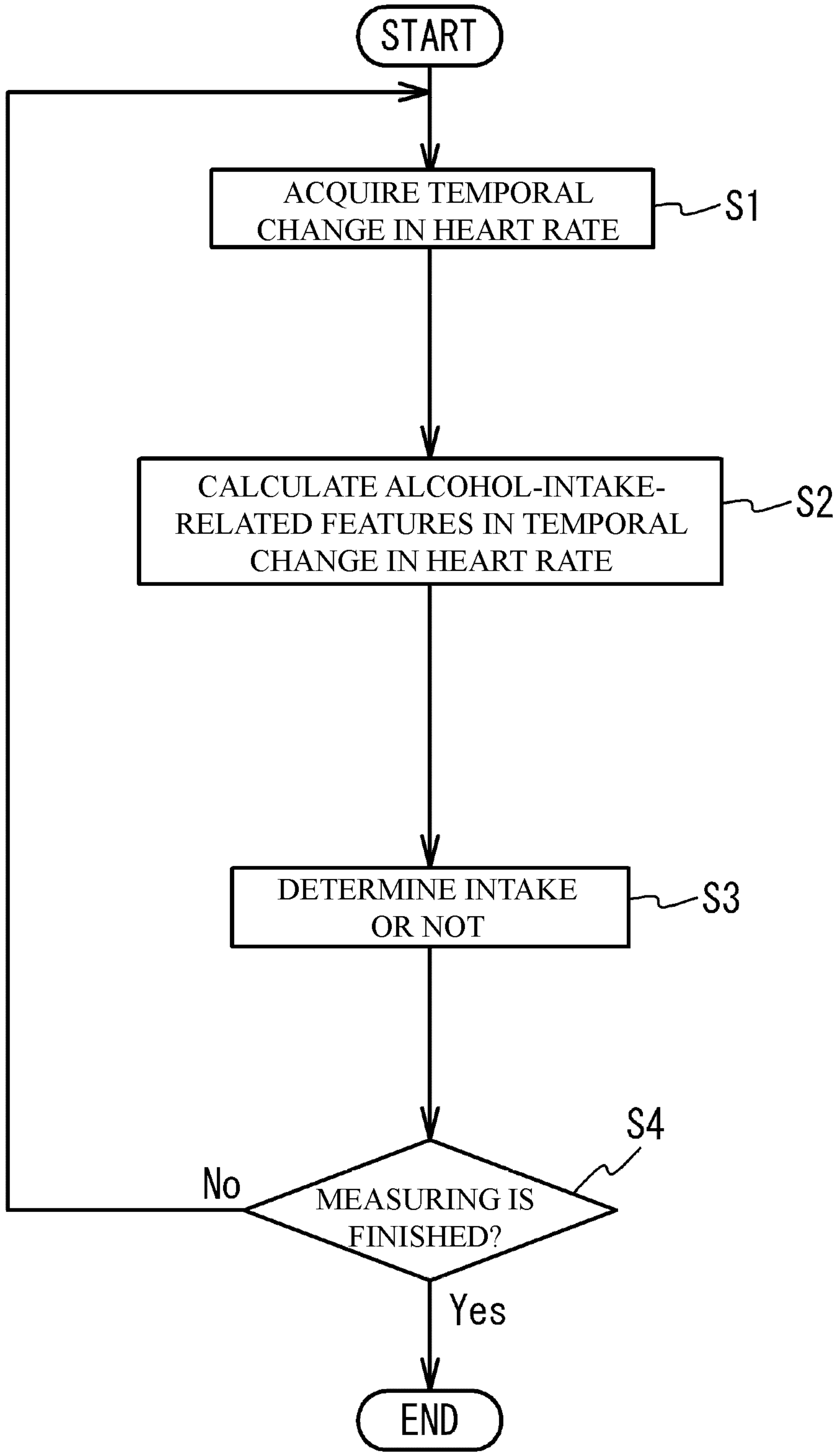
START
ACQUIRE TEMPORAL CHANGE IN HEART RATE
S1
CALCULATE ALCOHOL-INTAKE-RELATED FEATURES IN TEMPORAL CHANGE IN HEART RATE
S2
DETERMINE INTAKE OR NOT
S3
S4
No
MEASURING IS FINISHED?
Yes
END

FIG. 4

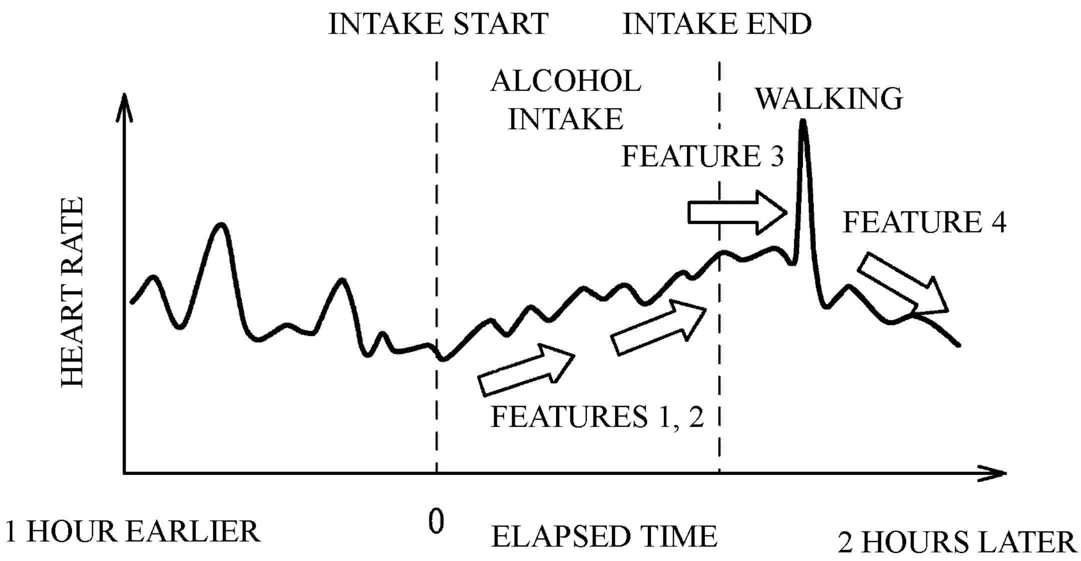
INTAKE START
INTAKE END
ALCOHOL INTAKE
WALKING
FEATURE 3
FEATURE 4
HEART RATE
FEATURES 1, 2
1 HOUR EARLIER
0
ELAPSED TIME
2 HOURS LATER

FIG. 5

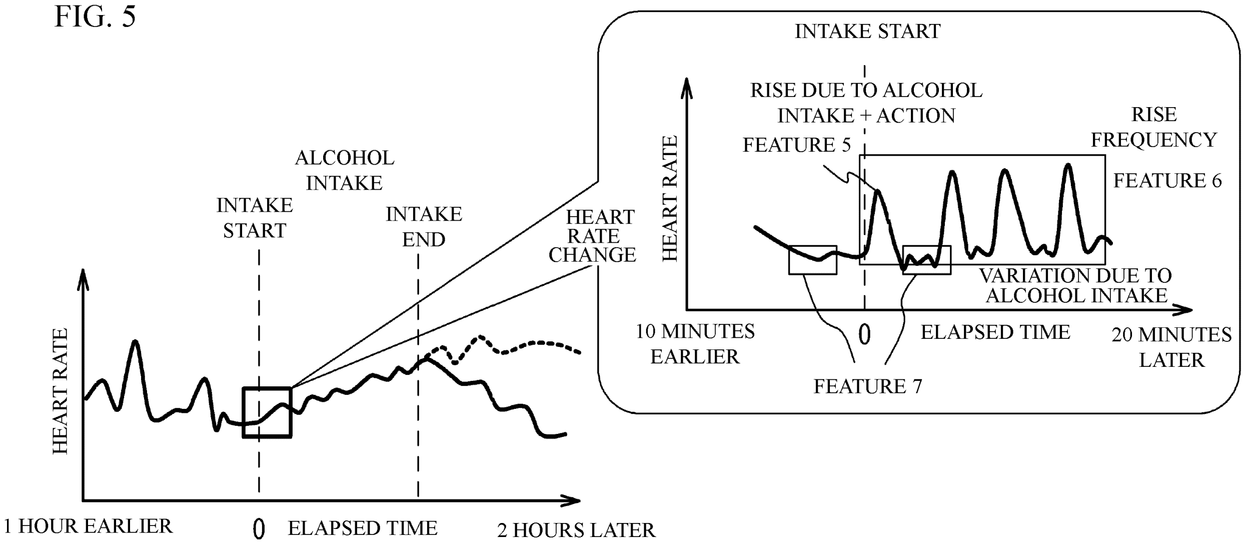
ALCOHOL INTAKE
INTAKE START
INTAKE END
HEART RATE CHANGE
HEART RATE
1 HOUR EARLIER
0
ELAPSED TIME
2 HOURS LATER
INTAKE START
RISE DUE TO ALCOHOL INTAKE + ACTION
FEATURE 5
RISE FREQUENCY
FEATURE 6
HEART RATE
VARIATION DUE TO ALCOHOL INTAKE
10 MINUTES EARLIER
0
ELAPSED TIME
20 MINUTES LATER
FEATURE 7

FIG. 6

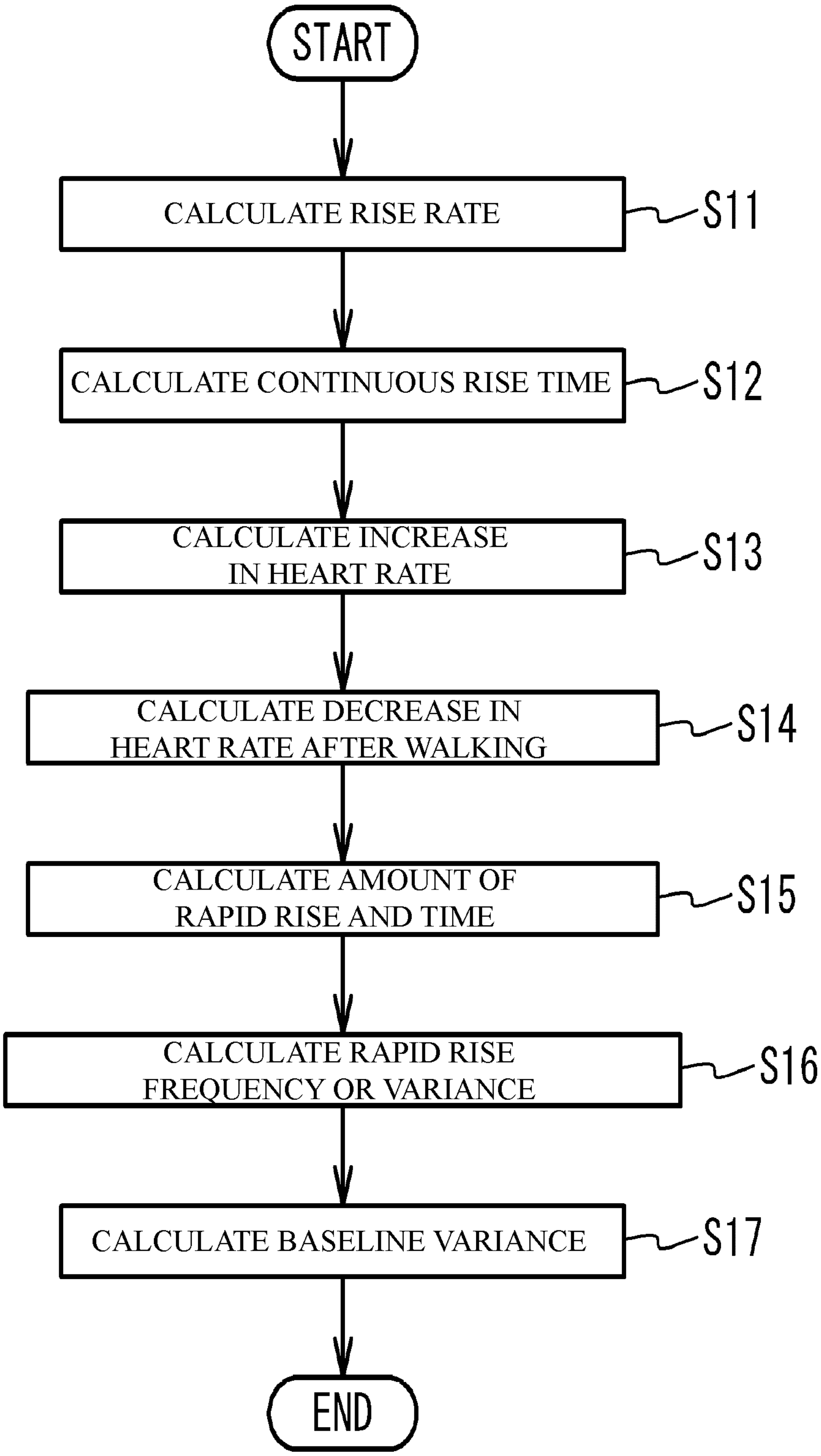
START
CALCULATE RISE RATE
S11
CALCULATE CONTINUOUS RISE TIME
S12
CALCULATE INCREASE IN HEART RATE
S13
CALCULATE DECREASE IN HEART RATE AFTER WALKING
S14
CALCULATE AMOUNT OF RAPID RISE AND TIME
S15
CALCULATE RAPID RISE FREQUENCY OR VARIANCE
S16
CALCULATE BASELINE VARIANCE
S17
END

FIG. 7A

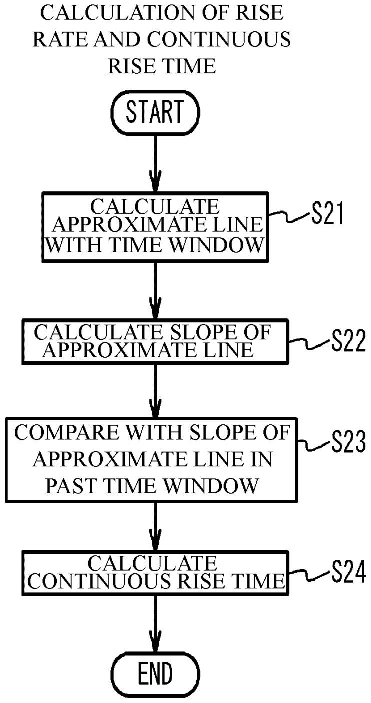


FIG. 7B

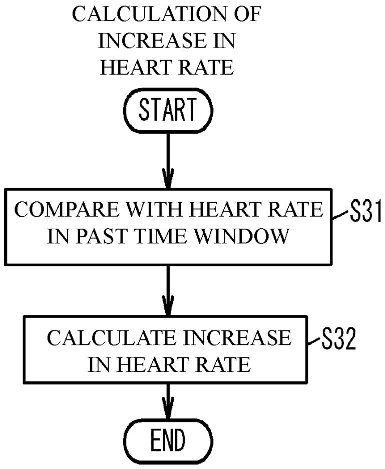


FIG. 7C

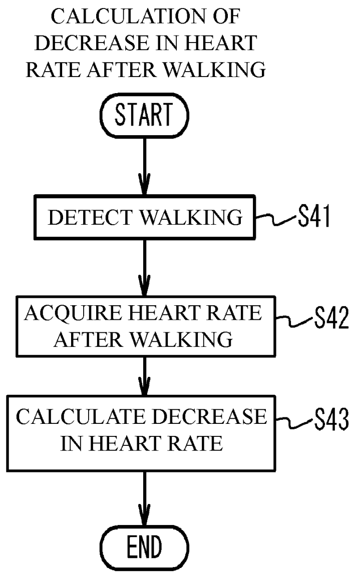

FIG. 8A

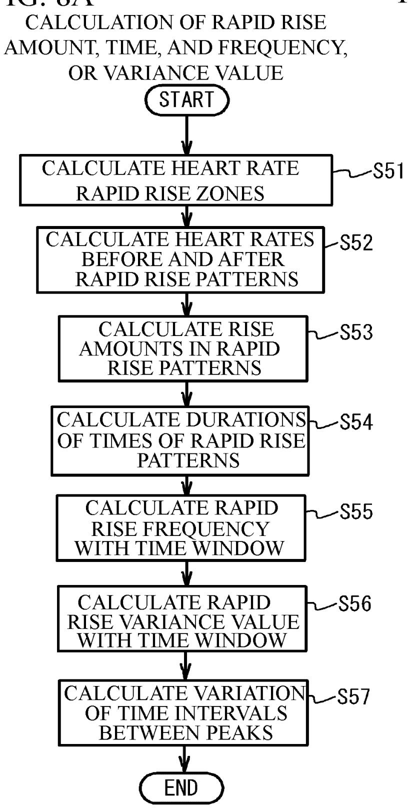


FIG. 8B

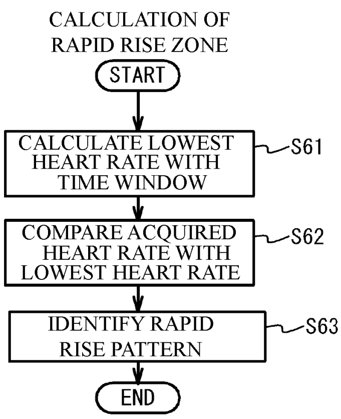


FIG. 8C

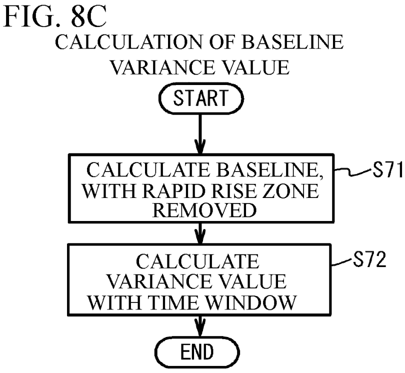

FIG. 9

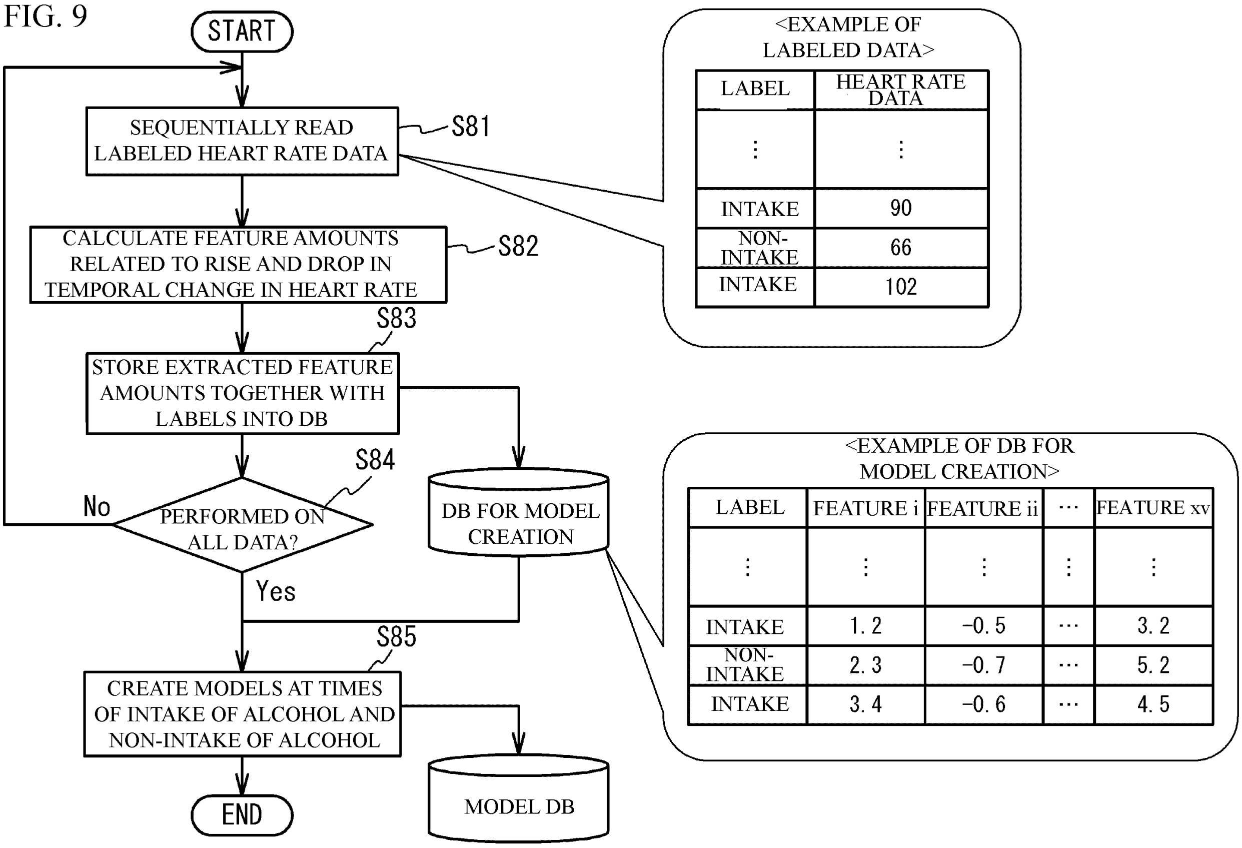
START
SEQUENTIALLY READ LABELED HEART RATE DATA
S81
CALCULATE FEATURE AMOUNTS RELATED TO RISE AND DROP IN TEMPORAL CHANGE IN HEART RATE
S82
S83
STORE EXTRACTED FEATURE AMOUNTS TOGETHER WITH LABELS INTO DB
S84
No
PERFORMED ON ALL DATA?
Yes
DB FOR MODEL CREATION
S85
CREATE MODELS AT TIMES OF INTAKE OF ALCOHOL AND NON-INTAKE OF ALCOHOL
END
MODEL DB
<EXAMPLE OF LABELED DATA>
LABEL
HEART RATE DATA
⋮
⋮
INTAKE
90
NON-INTAKE
66
INTAKE
102
<EXAMPLE OF DB FOR MODEL CREATION>
LABEL
FEATURE i
FEATURE ii
⋯
FEATURE xv
⋮
INTAKE
1. 2
−0. 5
⋯
3. 2
NON-INTAKE
2. 3
−0. 7
⋯
5. 2
INTAKE
3. 4
−0. 6
⋯
4. 5

FIG. 10

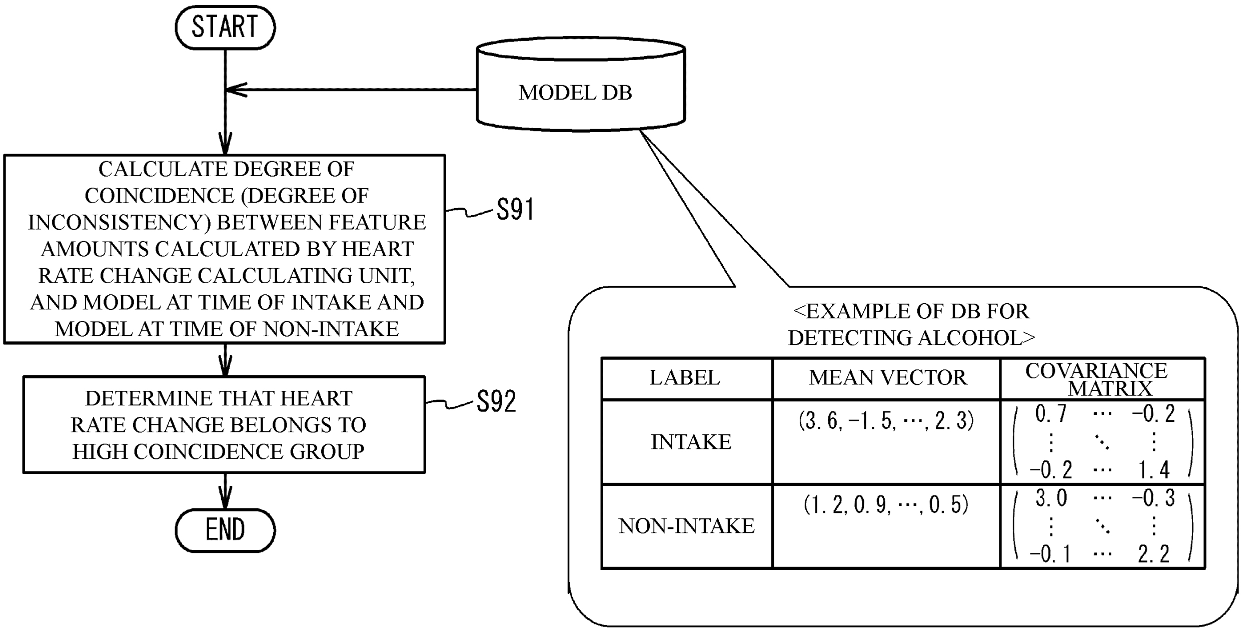
START
MODEL DB
CALCULATE DEGREE OF COINCIDENCE (DEGREE OF INCONSISTENCY) BETWEEN FEATURE AMOUNTS CALCULATED BY HEART RATE CHANGE CALCULATING UNIT, AND MODEL AT TIME OF INTAKE AND MODEL AT TIME OF NON-INTAKE
S91
DETERMINE THAT HEART RATE CHANGE BELONGS TO HIGH COINCIDENCE GROUP
S92
END
<EXAMPLE OF DB FOR DETECTING ALCOHOL>
LABEL
MEAN VECTOR
COVARIANCE MATRIX
INTAKE
(3. 6, −1. 5, ···, 2. 3)
0. 7 ··· −0. 2 ; −0. 2 ··· 1. 4
NON-INTAKE
(1. 2, 0. 9, ···, 0. 5)
3. 0 ··· −0. 3 ; −0. 1 ··· 2. 2

FIG. 11

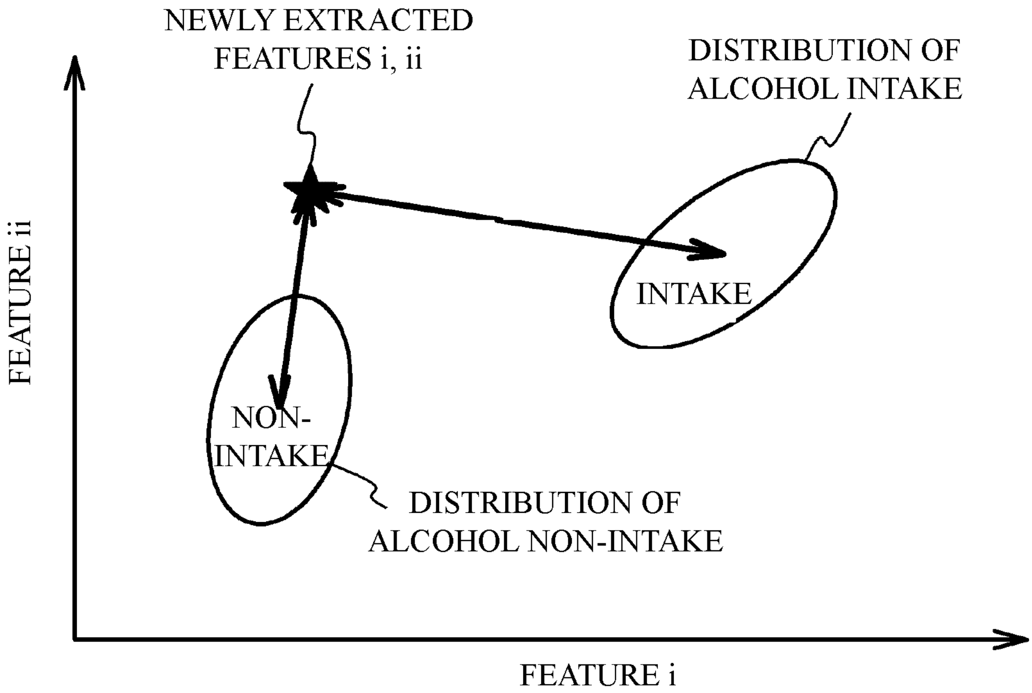
NEWLY EXTRACTED
FEATURES i, ii
DISTRIBUTION OF
ALCOHOL INTAKE
FEATURE ii
INTAKE
NON-
INTAKE
DISTRIBUTION OF
ALCOHOL NON-INTAKE
FEATURE i

FIG. 12

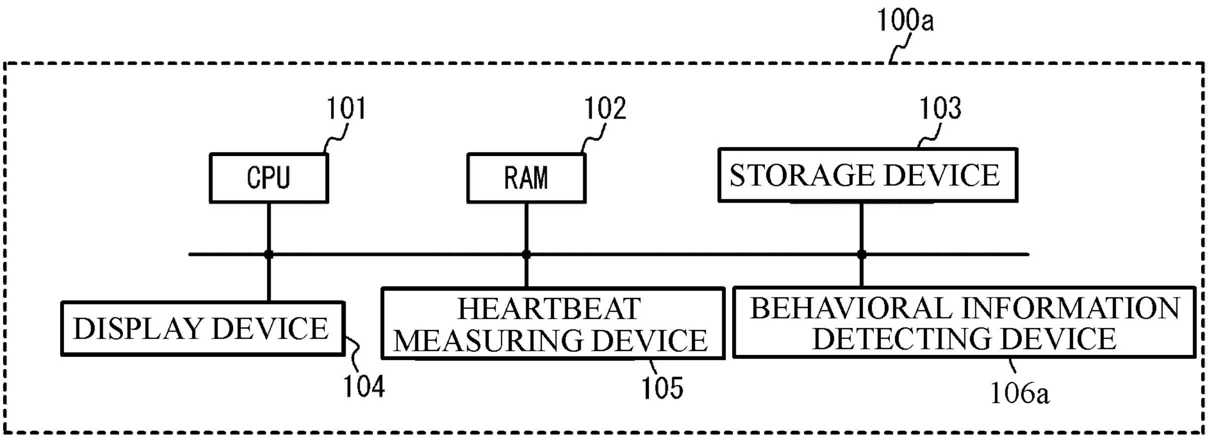
100a
101
102
103
CPU
RAM
STORAGE DEVICE
DISPLAY DEVICE
HEARTBEAT MEASURING DEVICE
BEHAVIORAL INFORMATION DETECTING DEVICE
104
105
106a

FIG. 13

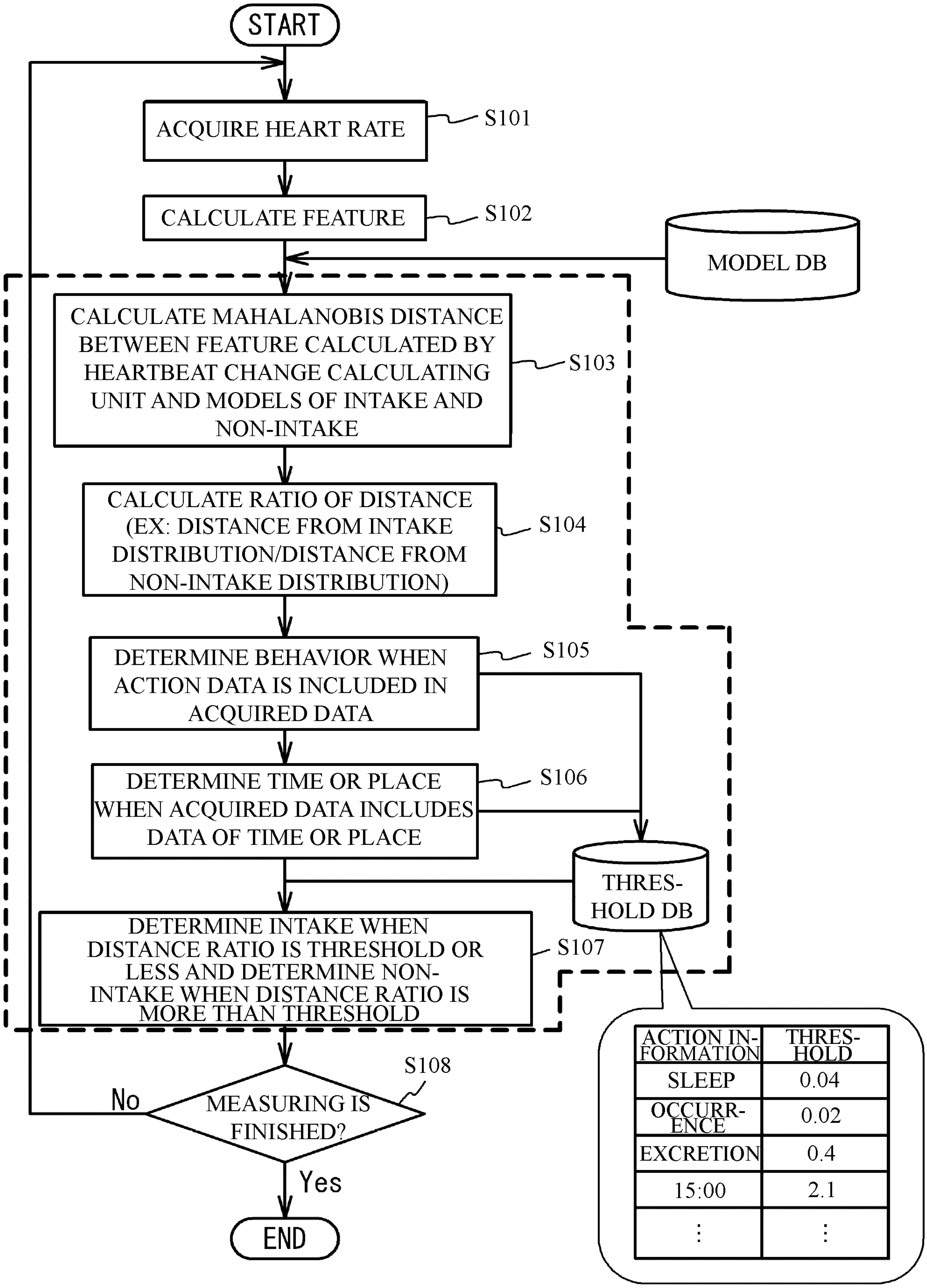
START
ACQUIRE HEART RATE
S101
CALCULATE FEATURE
S102
MODEL DB
CALCULATE MAHALANOBIS DISTANCE BETWEEN FEATURE CALCULATED BY HEARTBEAT CHANGE CALCULATING UNIT AND MODELS OF INTAKE AND NON-INTAKE
S103
CALCULATE RATIO OF DISTANCE (EX: DISTANCE FROM INTAKE DISTRIBUTION/DISTANCE FROM NON-INTAKE DISTRIBUTION)
S104
DETERMINE BEHAVIOR WHEN ACTION DATA IS INCLUDED IN ACQUIRED DATA
S105
DETERMINE TIME OR PLACE WHEN ACQUIRED DATA INCLUDES DATA OF TIME OR PLACE
S106
THRES-HOLD DB
DETERMINE INTAKE WHEN DISTANCE RATIO IS THRESHOLD OR LESS AND DETERMINE NON-INTAKE WHEN DISTANCE RATIO IS MORE THAN THRESHOLD
S107
ACTION IN-FORMATION
THRES-HOLD
SLEEP
0.04
OCCURR-ENCE
0.02
EXCRETION
0.4
15:00
2.1
⋮
⋮
S108
MEASURING IS FINISHED?
No
Yes
END

100b

FIG.14A

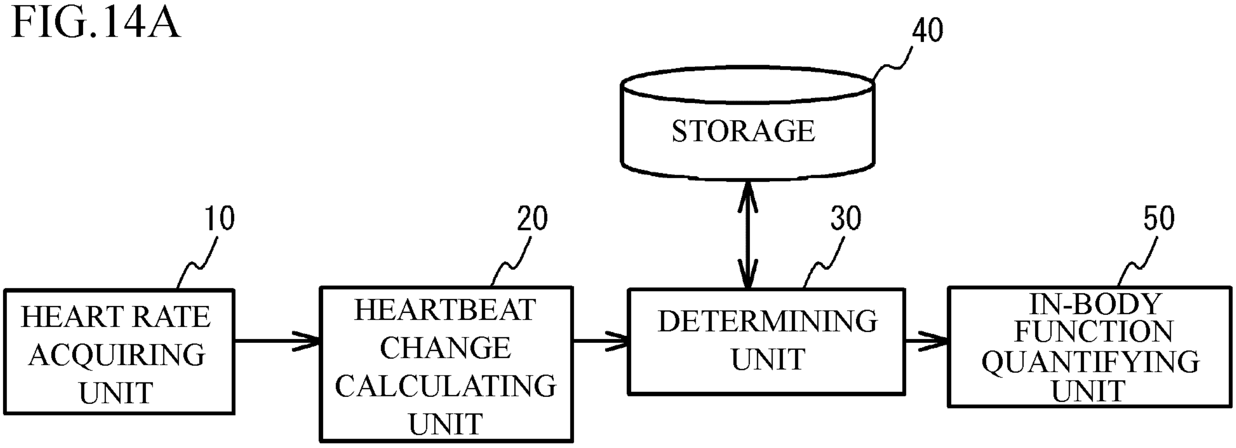


FIG. 14B

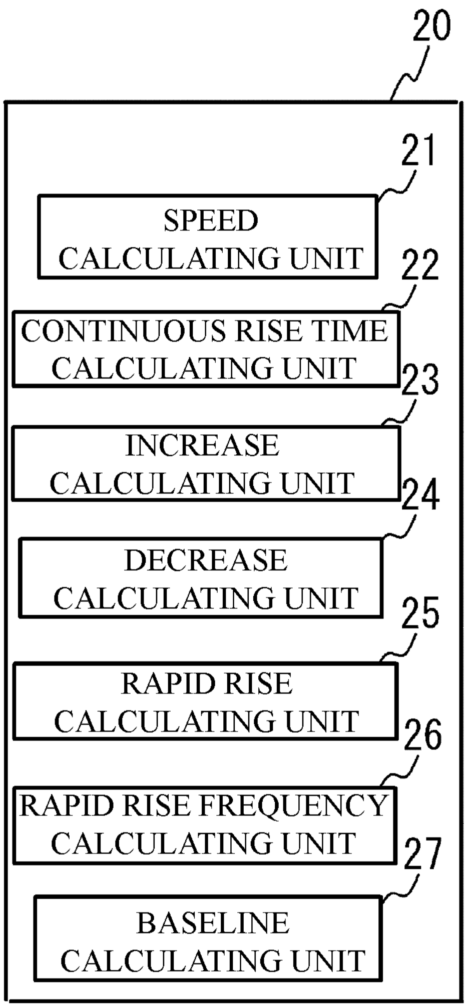

FIG. 15

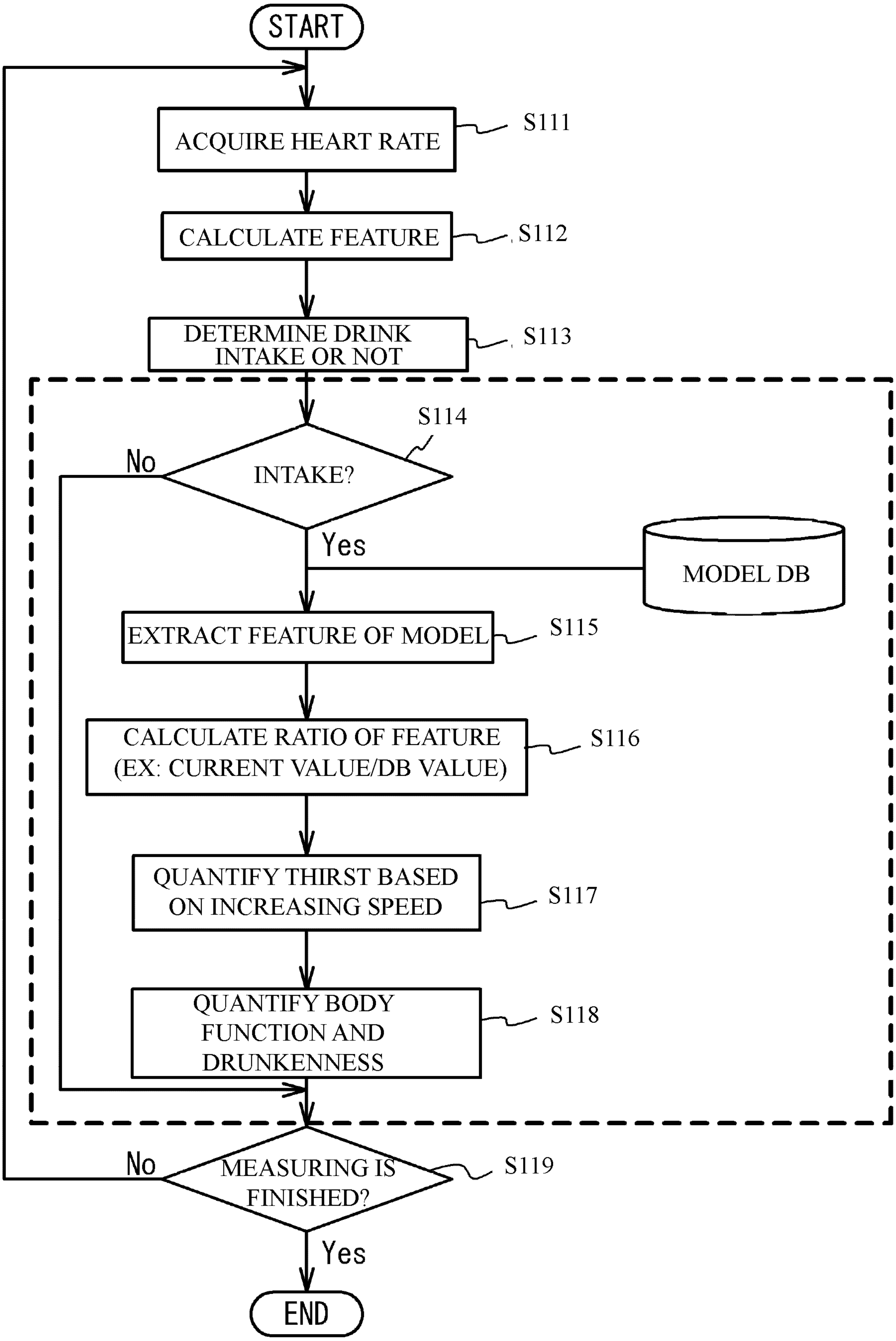
START
ACQUIRE HEART RATE
S111
CALCULATE FEATURE
S112
DETERMINE DRINK INTAKE OR NOT
S113
S114
INTAKE?
No
Yes
MODEL DB
EXTRACT FEATURE OF MODEL
S115
CALCULATE RATIO OF FEATURE (EX: CURRENT VALUE/DB VALUE)
S116
QUANTIFY THIRST BASED ON INCREASING SPEED
S117
QUANTIFY BODY FUNCTION AND DRUNKENNESS
S118
MEASURING IS FINISHED?
S119
No
Yes
END

FIG. 16A

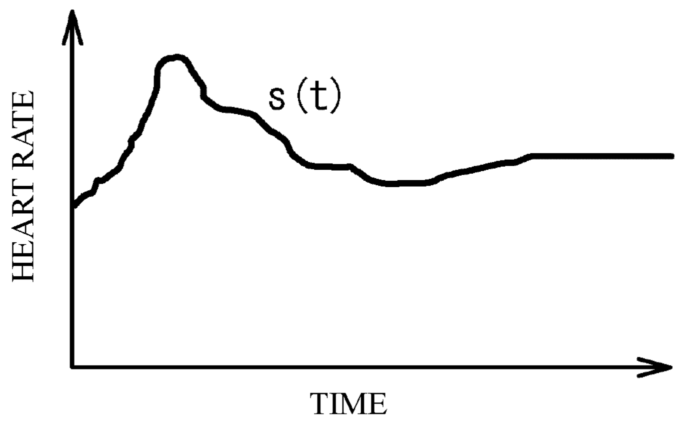


| s(1) | s(2) | ··· | s(N) |
|---|---|---|---|
| 50 | 58 | | 62 |

FIG. 16B

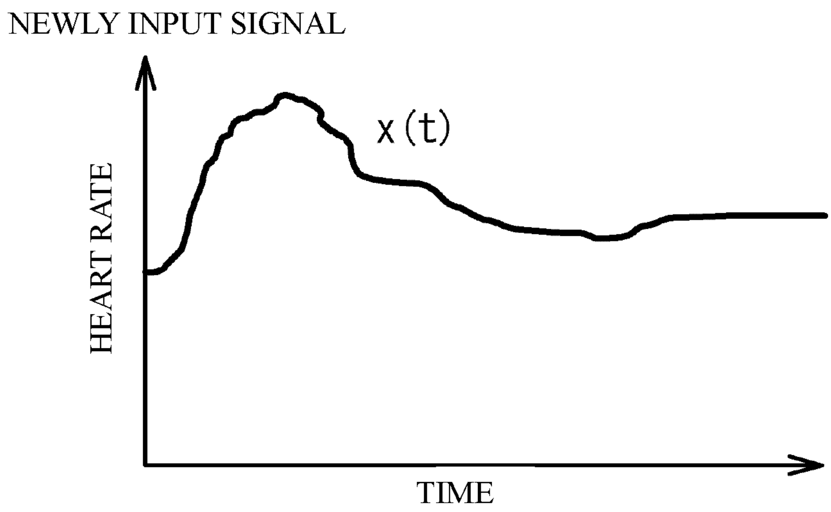


| x(1) | x(2) | ··· | x(N) |
|---|---|---|---|
| 52 | 53 | | 68 |

FIG. 17A

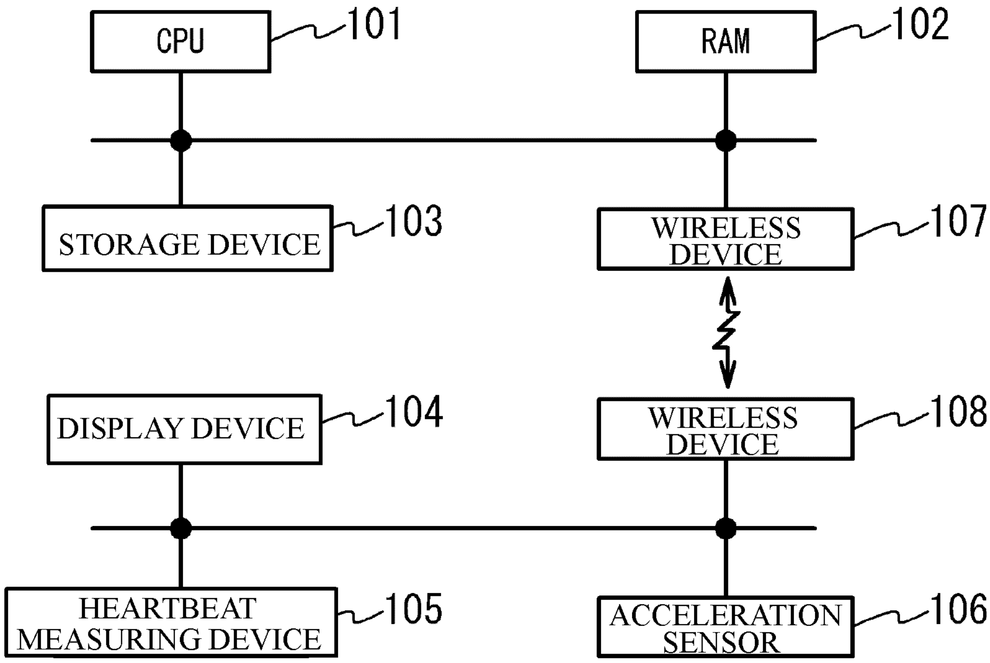


FIG. 17B

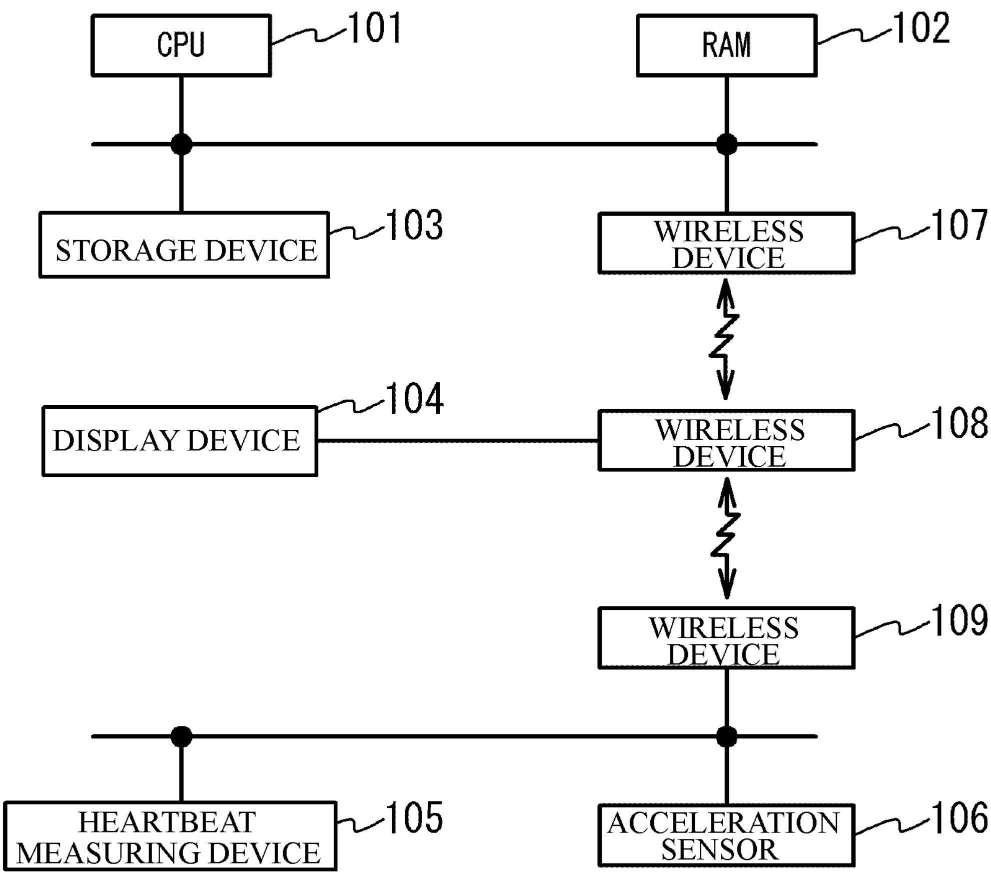

## INTERNATIONAL SEARCH REPORT

International application No.
PCT/JP2015/081859

A. CLASSIFICATION OF SUBJECT MATTER
*A61B5/025*(2006.01)i

According to International Patent Classification (IPC) or to both national classification and IPC

B. FIELDS SEARCHED

Minimum documentation searched (classification system followed by classification symbols)
A61B5/025

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 2009-219541 A (Toyota Motor Corp.), 01 October 2009 (01.10.2009), paragraphs [0016] to [0080]; fig. 1 to 12 (Family: none) | 1,6,17-18<br>2-5,7-16 |
| X<br>A | WO 2013/077253 A1 (Delta Tooling Co., Ltd.), 30 May 2013 (30.05.2013), paragraphs [0027] to [0092]; fig. 1 to 25 & JP 2013-111103 A & US 2014/0371603 A1 paragraphs [0252] to [0325]; fig. 1 to 25 & EP 2783631 A1 | 1,6,17-18<br>2-5,7-16 |
| A | JP 9-168516 A (Casio Computer Co., Ltd.), 30 June 1997 (30.06.1997), (Family: none) | 1-18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>20 January 2016 (20.01.16) | Date of mailing of the international search report<br>02 February 2016 (02.02.16) |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

International application No.
PCT/JP2015/081859

C (Continuation). DOCUMENTS CONSIDERED TO BE RELEVANT

| Category* | Citation of document, with indication, where appropriate, of the relevant passages | Relevant to claim No. |
|---|---|---|
| A | JP 2004-242720 A (Kizakura Sake Brewing Co.), 02 September 2004 (02.09.2004), (Family: none) | 1-18 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010134525 A